# EUROPEAN PATENT SPECIFICATION

(11) **EP 3 713 952 B1**
(45) Date of publication and mention of the grant of the patent: **05.01.2022**
(21) Application number: 18827226.4
(22) Date of filing: 22.11.2018
(51) Int. Cl.: C07K 5/02

(54) **METHOD FOR THE SYNTHESIS OF GLUTATHIONE DERIVATIVES**
VERFAHREN ZUR SYNTHESE VON GLUTATHIONDERIVATEN
PROCÉDÉ DE SYNTHÈSE DE DÉRIVÉS DE DE GLUTATHION

(30) Priority: 22.11.2017 IT 201700133856
(43) Date of publication of application: 30.09.2020
(73) Proprietor: Redox-co S.r.l., 00162 Roma (IT)
(72) Inventor: BARTOCCINI, Francesca, 61029 Urbino (PU) (IT); FRATERNALE, Alessandra, 61029 Urbino (PU) (IT); PIERSANTI, Giovanni, 61029 Urbino (PU) (IT)
(74) Representative: Bosman, Cesare
(86) International application number: PCT/IB2018/059234
(87) International publication number: WO 2019/102397

(56) References cited:
- JP-A- S 632 922
- PALAMARA A T ET AL: "NEW SYNTHETIC GLUTATHIONE DERIVATIVES WITH INCREASED ANTIVIRAL ACTIVITIES", ANTIVIRAL CHEMISTRY & CHEMOTHER, INTERNATIONAL MEDICAL PRESS, GB, vol. 15, no. 2, 1 March 2004 (2004-03-01), pages 83-91, XP009045775, ISSN: 0956-3202
- ALAN KATRITZKY ET AL: "Efficient and Selective Syntheses of S-Acyl and N-Acyl Glutathiones", SYNLETT, vol. 2010, no. 09, 16 April 2010 (2010-04-16), pages 1337-1340, XP055281907, DE ISSN: 0936-5214, DOI: 10.1055/s-0029-1219837

## Description

### CROSS-REFERENCE TO RELATED APPLICATIONS

This patent application claims priority from Italian patent application no. 102017000133856 filed on 22/11/2017.

### TECHNICAL FIELD

The present invention concerns a method for the synthesis of glutathione derivatives.

### BACKGROUND ART

Glutathione is undoubtedly one of the most important antioxidant and detoxifying agents which the organism is able to produce and plays an important role in the immune response. At a cellular level it is synthesized from glutamate, from glycine and from cysteine, which is the donor of the thiol group (-SH) responsible for the biological activity of glutathione.

Numerous physiopathological conditions (ageing, degenerative diseases, etc.) are characterized by oxidative stress and depletion of glutathione. In these states it is certainly advantageous to restore the physiological levels of glutathione in the tissues. However, the intake of glutathione from external sources is not particularly effective, since it lacks a specific carrier allowing it to cross the cell membrane.

It has been found that the glutathione derivative that best guarantees crossing of the cell membrane, at the same time maintaining the antioxidant and detoxifying characteristics of the glutathione, is N-acyl glutathione.

So far, the synthesis methods used all have criticalities in production terms such as to jeopardize their use on a large scale. In this regard, one of the synthesis methods proposed entails N-acylation of the glutathione with activated ester followed by a reduction. However, in this method other susceptible groups are present in the molecule during the reduction.

Another synthesis method entails use of the trityl group to protect the thiol group of the glutathione, then proceeding with the N-acylation and subsequent deprotection of the trityl group. As will be immediately evident to a person skilled in the art, this method involves timescales, costs and yields that prevent its application on an industrial scale.

Another method entails the use of 4-nitrobenzoyl to protect the thiol group of the glutathione, then proceeding with acylation of the NH₂ group with 1-acyl-1H-benzotriazole and subsequent deprotection, using pyrrolidine in dry methanol for four hours. Here again, timescales, costs and yields prevent the use of the method on a large scale.

Another synthesis method (disclosed in JP S63 2922) entails use of the Boc-group to protect the thiol group of the glutathione, then proceeding with the N-acylation and subsequent deprotection of the Boc-group.

The need was therefore felt for a method for synthesis of the N-acyl glutathione with technical characteristics such as to guarantee advantageous application thereof on an industrial scale.

### DISCLOSURE OF INVENTION

The subject of the present invention is a synthesis method for the preparation of N-acyl glutathione characterized in that it comprises an acylation step in which, in a water solution with pH ranging from 8 to 10, the glutathione is caused to react with an anhydride with formula (RCO)₂O with the formation of S,N-diacyl glutathione, and a subsequent selective alcoholysis step of the S-acyl group, in which S,N-diacyl glutathione produced in the preceding step is dissolved in an alkoxide/alcohol (R'O⁻/R'OH) solution and subsequently the product obtained is treated with a cation-exchange resin; R being H or a straight or branched hydrocarbon group with a number of carbon atoms ranging from C1 to C24; R' being a straight or branched radical hydrocarbon group with a number of carbon atoms ranging from C1 to C4.

Preferably, in said acylation step the water solution comprises a carbonate of an alkaline or alkaline earth metal or a bicarbonate of an alkaline metal.

Preferably, R is chosen from the group composed of a straight or branched C1-C24 alkyl group, a straight or branched C2-C24 alkenyl group, a C6-C24 aryl group, a straight or branched group with a C6-C14 aryl portion and a C1-C6 alkyl portion, a mixed C6-C14 aryl and C1-C6 alkyl or alkenyl group; said aryl group having 0 to more equal or different substituents which are comprised in the group consisting of atom of halogen, hydroxy, straight or branched, saturated or unsaturated C1-C6 alkoxide, amino, possibly monosubstituted or polysubstituted, substituted with a straight or branched C1-C6 alkyl group.

Preferably, R' is CH3.

Preferably, said acylation step comprises a solution acidification operation in which the glutathione is caused to react with an anhydride with formula (RCO)₂O.

Preferably, said acylation step and said selective alcoholysis step are carried out at room temperature.

### BEST MODE FOR CARRYING OUT THE INVENTION

Some embodiment examples are given below purely by way of nonlimiting example.

### - Acylation step -

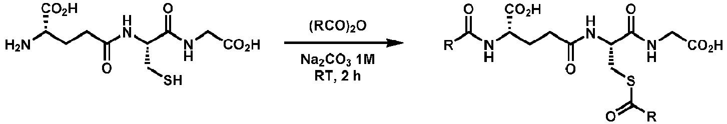

A 1M water solution of Na₂CO₃ in which 100 mg of glutathione are dissolved was placed in a three-necked flask. To this solution 0.668 mmoles of acetic anhydride ((CH₃CO)₂O) were added dropwise and the solution thus produced was kept under stirring for two hours. Subsequently, the solution was acidified to pH 3.5 by the addition of approximately 0.2 mL of a 6N solution of HCl. The water phase was washed three times with 3 mL of ethyl ether and then acidified to pH 1.5 by the addition of approximately 0.1 mL of a 6N solution of HCl. Subsequently, the water solution was concentrated at reduced pressure up to half volume and then decanted.

The same procedure as above was repeated using an anhydride different from acetic anhydride.

In particular, the procedure was repeated using butanoic anhydride ((CH₃CH₂CH₂CO)₂O), 2,2-dimethyl propionic anhydride ((CH₃CH₃ CH₃CCO)₂O) and benzoic anhydride ((PhCO)₂O) respectively.

The compounds S,N-diacyl glutathione produced underwent spectroscopic analysis to verify their identity.

The mass spectroscopy and magnetic resonance values obtained for each of the four S,N-diacyl glutathiones prepared are reported below:
R = Me
Yield: 77%
MS (ESI): 392 (M+1)+.

1H NMR (400 MHz, MeOH-d4) δ 4.60 (dd, J1 = 8.5 and J2 = 5.0 Hz, 1H), 4.30 (dd, J1 = 8.0 and J2 = 5.0 Hz, 1H), 3.85 (d, J = 17.0 Hz, 1H), 3.75 (d, J = 17.0 Hz, 1H), 3.48 (dd, J1 = 14.0 and J2 = 5.0 Hz, 1H), 3.15 (dd, J1 = 14.0 and J2 = 8.5 Hz, 1H), 2.37-2.33 (m, 5H), 2.20-2.11 (m, 1H), 2.03-1.96 (m, 4H).

13C NMR (100 MHz, MeOH-d4) δ 195.7, 176.1, 174.1, 173.9, 171.5, 170.6, 53.6, 52.7, 42.5, 31.9, 30.2, 29.0, 28.0, 21.4.
R = n-Pr
Yield: 85%
MS (ESI): 448 (M+1)+.

1H NMR (400 MHz, D2O) δ 4.62 (dd, J1 = 8.0 and J2 = 5.0 Hz, 1H), 4.32 (dd, J1 = 9.0 and J2 = 5.0 Hz, 1H), 3.96 (s, 2H), 3.42 (dd, J1 = 14.5 and J2 = 5.0 Hz, 1H), 3.13 (dd, J1 = 14.5 and J2 = 8.0 Hz, 1H), 2.59 (t, J = 7.0 Hz, 2H), 2.39 (t, J = 7.5 Hz, 2H), 2.24 (t, J = 7.0 Hz, 2H), 2.19-2.12 (m, 1H), 2.00-1.91 (m, 1H), 1.65-1.54 (m, 4H), 0.88 (t, J = 7.0, 3H), 0.87 (t, J = 7.0, 3H).

13C NMR (100 MHz, D2O) δ 203.7, 177.3, 175.3, 174.9, 173.0, 172.1, 52.6, 52.1, 52.1, 45.3, 41.2, 37.3, 31.6, 29.9, 26.3, 18.9, 12.7, 12.6.
R = t-Bu
Yield: 83%
MS (ESI): 476 (M+1)+.

1H NMR (400 MHz, MeOH-d4) δ 4.57 (dd, J1 = 9.0 and J2 = 5.0 Hz, 1H), 4.26 (dd, J1 = 7.0 and J2 = 5.5 Hz, 1H), 3.84 (d, J = 17.0 Hz, 1H), 3.73 (d, J = 17.0 Hz, 1H), 3.46 (dd, J1 = 14.0 and J2 = 5.0 Hz, 1H), 3.13 (dd, J1 = 14.0 and J2 = 9.0 Hz, 1H), 2.32 (t, J = 7.5 Hz, 2H), 2.17-2.02 (m, 2H), 1.24 (s, 9H), 1.23 (s, 9H).

13C NMR (100 MHz, MeOH-d4) δ 206.2, 179.1, 176.4, 174.2, 174.1, 170.6, 53.8, 52.8, 47.0, 46.1, 42.8, 31.8, 29.7, 28.2, 26.4, 26.3.
R = Ph
Yield: 72%
MS (ESI): 516 (M+1)+.

1H NMR (400 MHz, MeOH-d4) δ 7.94 (d, J = 7.5 Hz, 2H), 7.86 (d, J = 7.5 Hz, 2H), 7.64-7.44 (m, 6H), 4.76 (dd, J1 = 8.5 and J2 = 5.0 Hz, 1H), 4.59 (dd, J1 = 8.5 and J2 = 5.0 Hz, 1H), 3.90 (d, J = 17.0 Hz, 1H), 3.83 (d, J = 17.0 Hz, 1H), 3.70 (dd, J1 = 14.0 and J2 = 5.0 Hz, 1H), 3.36 (dd, J1 = 14.0 and J2 = 9.0 Hz, 1H), 2.46 (t, J = 7.5 Hz, 2H), 2.36-2.27 (m, 1H), 2.19-2.10 (m, 1H).

13C NMR (100 MHz, MeOH-d4) δ 189.8, 173.1, 172.5, 171.0, 169.3, 167.0, 135.1, 131.8, 129.4, 126.9, 126.6, 126.5, 125.5, 125.3, 51.2, 46.9, 39.9, 30.4, 28.5, 25.7

From the values reported above, the nature of the S,N-acylate compounds with the RCO groups deriving from the respective anhydrides used are ascertained.

### - Selective alcoholysis step -

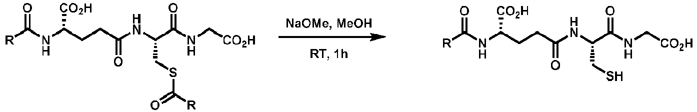

A solution in methanol of 0.31 mmol of S,N-diacyl glutathione prepared as described above was placed in a 10mL flask equipped with a magnetic anchor. 0.93 mL of a 1 M solution of sodium methoxide in methanol were added to the solution. The solution was kept under stirring for one hour at room temperature and then the solvent was evaporated at reduced pressure.

The solid residue obtained was solubilized in 1 mL of water and poured slowly into a beaker containing a cation-exchange resin (Dowex 50W-X8(H)) and 0.4 mL of water.

In particular, before use the resin was regenerated by means of a 0.25N solution of H₂SO₄ and subsequently washed with water until detecting an absence of sulphate and a pH equal to 7.

The suspension in the beaker was filtered and the resin was washed with 5 mL of water. The filtrate was concentrated at reduced pressure by means of coevaporation with 1 mL of xylene. The solid obtained was ground with 5 mL of ethyl ether and filtered.

The respective N-acyl glutathione obtained underwent spectroscopic analysis to verify its identity.

The mass spectroscopy and magnetic resonance values obtained for each of the four N-acyl glutathiones prepared are reported below:
R = Me
Yield: 75%
MS (ESI): 350 (M+1)+.

1H NMR (400 MHz, MeOH-d4) δ 4.56 (dd, J1 = 7.0 and J2 = 4.5 Hz, 1H), 4.38 (dd, J1 = 8.5 and J2 = 5.0 Hz, 1H), 3.89 (s, 2H), 2.94-2.87 (m, 2H), 2.42 (t, J = 7.5 Hz, 2H), 2.24-2.16 (m, 1H), 2.10 - 1.94 (m, 4H).

13C NMR (100 MHz, MeOH-d4) δ 174.9, 173.8, 172.7, 171.8, 171.1, 55.6, 52.7, 41.5, 31.7, 27.5, 25.5, 21.2.
R = n-Pr
Yield: 91%
MS (ESI): 378 (M+1)+

1H NMR (400 MHz, D2O) δ 4.52 (dd, J1 = 7.0 and J2 = 5.5 Hz, 1H), 4.25 (dd, J1 = 9.0 and J2 = 5.0 Hz, 1H), 3.89 (s, 2H), 2.92-2.83 (m, 2H), 2.42 (t, J = 7.5 Hz, 2H), 2.23 (t, J = 7.5 Hz, 2H), 2.19 - 2.12 (m, 1H), 1.99-1.91 (m, 1H), 1.61-1.52 (m, 2H), 0.87 (t, J = 7.5Hz, 3H);

13C NMR (100 MHz, D2O) δ 177.2, 176.0, 175.3, 173.8, 172.3, 55.5, 52.6, 41.7, 37.4, 31.6, 26.5, 25.5, 18.9, 12.8.
R = t-Bu
Yield: 80%
MS (ESI): 392 (M+1)+.

1H NMR (400 MHz, MeOH-d4) δ 4.58 (dd, J1 = 7.0 and J2 = 5.5 Hz, 1H), 4.39 (dd, J1 = 9.0 and J2 = 5.0 Hz, 1H), 3.94 (s, 2H), 2.91-2.86 (m, 2H), 2.44 (t, J = 7.0 Hz, 2H), 2.29-2.21 (m, 1H), 2.09-2.00 (m, 1H), 1.23 (s, 9H).

13C NMR (100 MHz, MeOH-d4) δ 180.0, 174.1, 173.8, 171.6, 171.3, 55.5, 52.3, 40.7, 38.2, 31.6, 26.6, 26.4, 25.6.
R = Ph
Yield: 73%
MS (ESI): 412 (M+1)+.

1H NMR (400 MHz, MeOH-d4) δ 8.02 (d, J = 7.5 Hz, 2H), 7.47-7.42 (m, 3H), 4.63-4.56 (m, 2H), 3.87 (s, 2H), 2.92-2.81 (m, 2H), 2.55-2.48 (m, 2H), 2.36-2.30 (m, 1H), 2.19-2.10 (m, 1H). 13C NMR (100 MHz, MeOH-d4) δ 172.7, 172.3, 170.4, 169.6, 167.0, 130.9, 127.6, 126.5, 125.5, 53.9, 51.3, 39.4, 30.2, 25.3, 23.9.

From the above values the nature of the N-acylate products is ascertained with re-establishment of the SH group in the respective S,N-acyl glutathione substrate used.

From the above description it is immediately evident that the synthesis method subject of the present invention offers the great advantage of being extremely simple, inexpensive and guaranteeing a high yield. In fact, it should be highlighted that the acylation and selective alcoholysis reactions take place at room temperature, and that solvents are used such as water (for the acylation reaction) and methanol (for the selective alcoholysis reaction).

The above advantages mean that the synthesis method subject of the present invention can be applied on an industrial scale without suffering the productivity problems typical of the methods of the known art.

## Claims

1. A synthesis method for the production of N-acyl glutathione, **characterized in that** it comprises an acylation step, during which, in a water solution with a pH ranging from 8 to 10, glutathione is caused to react with an anhydride with formula (RCO)₂O with a formation of S,N-diacyl glutathione, and a subsequent S-acyl group selective alcoholysis step, during which the S,N-diacyl glutathione produced in the previous step is dissolved in an alkoxide/alcohol solution (R'O⁻/R'OH) and, subsequently, the obtained product is treated with a cation-exchange resin; R being H or a straight or branched hydrocarbon group with a number of carbon atoms ranging from C1 to C24; R' being a straight or branched radical hydrocarbon group with a number of carbon atoms ranging from C1 to C4.

2. The synthesis method according to claim 1, **characterized in that**, during said acylation step, the water solution comprises a carbonate of an alkaline or alkaline earth metal or a bicarbonate of an alkaline metal.

3. The synthesis method according to claim 1 or 2, **characterized in that** R is chosen in the group consisting of a straight or branched C1-C24 alkyl group, a straight or branched C2-C24 alkenyl group, a C6-C24 aryl group, a straight or branched group with a C6-C14 aryl portion and a C1-C6 alkyl portion, a mixed C6-C14 aryl and C1-C6 alkyl or alkenyl group; said aryl group having 0 to more equal or different substituents, which are comprised in the group consisting of atom of halogen, hydroxy, straight or branched, saturated or unsaturated C1-C6 alkoxide, amino, possibly monosubstituted or polysubstituted, substituted with a straight or branched C1-C6 alkyl group.

4. The synthesis method according to one of the preceding claims, **characterized in that** R' is CH3.

5. The synthesis method according to one of the preceding claims, **characterized in that** said acylation step comprises a solution acidification operation, during which glutathione is caused to react with an anhydride with formula (RCO)₂O.

6. The synthesis method according to one of the preceding claims, **characterized in that** said acylation step and said selective alcoholysis step are carried out at room temperature.

## Patentansprüche

1. Ein Syntheseverfahren zur Herstellung von N-Acyl-Glutathion, **dadurch gekennzeichnet, dass** es einen Acylierungsschritt umfasst, bei dem Glutathion in einer Wasserlösung mit einem pH-Wert im Bereich von 8 bis 10 mit einem Anhydrid mit der Formel (RCO)₂O unter Bildung von S,N-Diacylglutathion zur Reaktion gebracht wird, und einen anschließenden S-Acylgruppen-selektiven Alkoholyseschritt, bei dem das im vorhergehenden Schritt hergestellte S,N-Diacylglutathion in einer Alkoxid/AlkoholLösung (R'O⁻/R'OH) gelöst wird, und anschließend das erhaltene Produkt mit einem Kationenaustauscherharz behandelt wird; wobei R gleich H oder eine geradkettige oder verzweigte Kohlenwasserstoffgruppe mit einer Anzahl an Kohlenstoffatomen im Bereich von bis C24 ist; wobei R' ein Rest einer geradkettigen oder verzweigten Kohlenwasserstoffgruppe mit einer Anzahl an Kohlenstoffatomen im Bereich von bis C4 ist.

2. Das Syntheseverfahren nach Anspruch 1, **dadurch gekennzeichnet, dass** die Wasserlösung während des Acylierungsschritts ein Alkali- oder Erdalkalimetallcarbonat oder ein Alkalimetallbicarbonat enthält.

3. Das Syntheseverfahren nach Anspruch 1 oder 2, **dadurch gekennzeichnet, dass** R ausgewählt ist aus der Gruppe bestehend aus einer geradkettigen oder verzweigten C1-C24-Alkylgruppe, einer geradkettigen oder verzweigten C2-C24-Alkenylgruppe, einer C6-C24-Arylgruppe, einer geradkettigen oder verzweigten Gruppe mit einem C6-C14-Arylanteil und einem C1-C6-Alkylanteil, einer gemischten C6-C14-Aryl- und C1-C6-Alkyl- oder Alkenylgruppe; wobei die Arylgruppe 0 bis mehrere gleiche oder verschiedene Substituenten aufweist, die umfasst sind in der Gruppe bestehend aus Halogenatom, Hydroxy, geradkettigem oder verzweigtem, gesättigtem oder ungesättigtem C1-C6-Alkoxid, Amino, möglicherweise mono- oder polysubstituiert, substituiert mit einer geradkettigen oder verzweigten C1-C6-Alkylgruppe.

4. Das Syntheseverfahren nach einem der vorherigen Ansprüche, **dadurch gekennzeichnet, dass** R' gleich CH3 ist.

5. Das Syntheseverfahren nach einem der vorherigen Ansprüche, **dadurch gekennzeichnet, dass** der Acylierungsschritt einen Vorgang des Ansäuerns der Lösung umfasst, bei dem Glutathion mit einem Anhydrid der Formel (RCO)₂O zur Reaktion gebracht wird.

6. Das Syntheseverfahren nach einem der vorherigen Ansprüche, **dadurch gekennzeichnet, dass** der Acylierungsschritt und der selektive Alkoholyseschritt bei Raumtemperatur durchgeführt werden.

## Revendications

1. Méthode de synthèse pour la production de N-acyl glutathion, **caractérisée en ce qu'**elle comprend une étape d'acylation, pendant laquelle, dans une solution aqueuse ayant un pH dans la plage de 8 à 10, du glutathion est amené à réagir avec un anhydride de formule (RCO)₂O avec formation de S,N-diacyl glutathion, et une étape ultérieure d'alcoolyse sélective de groupe S-acyle, pendant laquelle le S,N-diacyl glutathion produit à l'étape précédente est dissous dans une solution d'alcoxyde/alcool (R'O⁻/R'OH) et, par la suite, le produit obtenu est traité avec une résine échangeuse de cations ; R étant H ou un groupe hydrocarboné linéaire ou ramifié ayant un nombre d'atomes de carbone dans la plage de C1 à C24 ; R' étant un groupe hydrocarboné à radical linéaire ou ramifié ayant un nombre d'atomes de carbone dans la plage de C1 à C4.

2. Méthode de synthèse selon la revendication 1, **caractérisée en ce que**, pendant ladite étape d'acylation, la solution aqueuse comprend un carbonate d'un métal alcalin ou alcalino-terreux ou un bicarbonate d'un métal alcalino-terreux.

3. Méthode de synthèse selon la revendication 1 ou 2, **caractérisée en ce que** R est sélectionné dans le groupe constitué d'un groupe C1-C24 alkyle linéaire ou ramifié, un groupe C2-C24 alcényle linéaire ou ramifié, un groupe C6-C24 aryle, un groupe linéaire ou ramifié avec une partie C6-C14 aryle et une partie C1-C6 alkyle, un groupe mixte C6-C14 aryle et C1-C6 alkyle ou alcényle ; ledit groupe aryle ayant de 0 à plus substituants identiques ou différents, qui sont compris dans le groupe constitué d'un halogène, un hydroxy, un C1-C6 alcoxyde linéaire ou ramifié, saturé ou insaturé, un amino, éventuellement monosubstitué ou polysubstitué, substitué par un groupe C1-C6 alkyle linéaire ou ramifié.

4. Méthode de synthèse selon l'une des revendications précédentes, **caractérisée en ce que** R' est CH3.

5. Méthode de synthèse selon l'une des revendications précédentes, **caractérisée en ce que** ladite étape d'acylation comprend une opération d'acidification de solution, pendant laquelle le glutathion est amené à réagir avec un anhydride de formule (RCO)₂O.

6. Méthode de synthèse selon l'une des revendications précédentes, **caractérisée en ce que** ladite étape d'acylation et ladite étape d'alcoolyse sélective sont réalisées à température ambiante.
